# EUROPEAN PATENT APPLICATION

(11) **EP 1 905 424 A2**
(43) Date of publication of application: **02.04.2008**
(21) Application number: 07101592.9
(22) Date of filing: 01.02.2007
(51) Int. Cl.: A61K 9/10, A61K 9/14, A61K 31/40

(54) **Pharmaceutical composition comprising stabilized statin particles**

(30) Priority: 02.02.2006 IN DE02832006
(71) Applicant: Ranbaxy Laboratories Limited, Gurgaon, Haryana 122001 (IN)
(72) Inventor: MATHUR, Rajeev Shankar, Gurgaon, 122002, Haryana (IN); Singh, Romi Barat, 221002, Varanasi Uttar Pradesh (IN); Aggarwal, Swati, Dwarka, 110045, Dehli (IN)
(74) Representative: Cronin, Brian Harold John

(57) **Abstract**

The present invention relates to stabilized statin particles and process for preparation thereof.

## Description

### Field of Invention

The present invention relates to stabilized statin particles and process for preparation thereof.

### Background of Invention

Statins are currently among the most therapeutically effective drugs available for reducing the level of LDL in the blood stream of a patient at risk for cardiovascular disease. Statins are also known to raise HDL cholesterol levels and decrease total triglyceride levels. It is believed that statins disrupt the biosynthesis of cholesterol and other sterols in the liver by competitively inhibiting the 3-hydroxy-3-methyl-glutaryl-coenzyme A reductase enzyme ("HMG-CoA reductase"). HMG-CoA reductase catalyzes the conversion of HMG-CoA to mevalonate, which is the rate determining step in the biosynthesis of cholesterol. Consequently, its inhibition leads to a reduction in the rate of formation of cholesterol in the liver.

The main statins currently used in therapeutics are: pravastatin, simvastatin, lovastatin, fluvastatin, atorvastatin and rosuvastatin. Lovastatin, simvastatin and pravastatin are fully or partially fermentation based products, whereas fluvastatin, rosuvastatin and atorvastatin are entirely synthetic. Simvastatin is a clinically modified 2,2-dimethyl-butyrate analogue of lovastatin. Pravastatin is a purified active metabolite of mevastatin with an open hydroxyacid instead of a lactone ring. All the statins are relatively unstable, and their degradation is catalyzed by several parameters like oxygen, humidity, acidity and temperature.

Pravastatin sodium (sold in the U.S. under the trade name PRAVACHOL^{®}) is sensitive to a low pH environment and degrades to form corresponding lactone and various isomers. U.S. Patent No. 5,180,589 discloses the use of one or more basifying agents to impart a desired pH of at least 9 to an aqueous dispersion of a pravastatin composition in order to stabilize it.

Atorvastatin calcium (sold in the U.S. under the trade name LIPITOR^{®}) is also susceptible to a low pH environment and can degrade to the corresponding lactone in an acidic environment. U.S. Patent No.. 5,686,104 discloses that atorvastatin and similar compounds in an oral pharmaceutical composition for the treatment of hypercholesterolemia or hyperlipidemia can be stabilized by combination with at least one basic inorganic pharmaceutically acceptable calcium, magnesium, aluminum or lithium salt.

The U.S. Patent No. 5,356,896 describes a pharmaceutical dosage form comprising an HMG-CoA reductase inhibitor compound, e.g. fluvastatin sodium, which is stabilized against pH-related degradation by an alkaline stabilizing medium capable of maintaining a pH of at least 8 to an aqueous solution or dispersion of the composition.

These statins are known to occur in various crystalline forms as well as amorphous forms. The use of amorphous form as the active substance is advantageous over crystalline form because the amorphous form dissolves faster and better which is an important factor for bioavailability of the active substance in the body. However, the stability of an active substance can depend on its polymorphic form. Amorphous forms can be none susceptible to oxidation, heat, light, moisture and low pH, as compared to crystalline forms. These factors can play a role in the stability of a pharmaceutical composition comprising an amorphous substance. Impurities generated upon degradation of active substances can reduce the therapeutic effects of an active substance and additionally unnecessarily burden the body with unnecessary degradation products. For examples, oxidative degradation of atorvastatin can lead to impurities such as Atorvastatin diepoxide, dihydroxy epoxide and diketoepoxide.

There have been various attempts to stabilize the amorphous statins. One of the approaches followed is use of inert gas or controlled atmospheric conditions during the process of packaging of the dosage form as disclosed in WO 04/032920 and WO 05/011638.

U.S. 2005/0106243 and U.S. 2005/0032880 disclose a method of producing amorphous atorvastatin particles, however they do not describe any method of stabilization of these particles.

Therefore, there is a constant need for preparing a stable pharmaceutical composition comprising amorphous statin.

### Summary of the Invention

It has herein been found that stability of statins can be achieved by one or more of the following techniques:
a) Layering an amorphous statin with stabilizers
b) Statin and stabilizer layering on inert carrier
   - By spraying a dispersion of amorphous statin in a solution of stabilizers
   - By separately spraying stabilizer solution and amorphous statin in dry form
c) Use of approach similar to (b) using crystalline API by converting crystalline material to amorphous material during processing (e.g. by using a solution of crystalline statin for spraying)

In one general aspect, there are provided stabilized statin particles comprising amorphous statin and stabilizers, wherein the statin is coated with stabilizers.

In another general aspect, there are provided processes for the preparation of stabilized statin particles comprising:
i) dissolving/dispersing one or more stabilizers in solvent;
ii) spraying the solution/dispersion of stabilizers onto the amorphous statin; and
iii) removing the solvent.

In yet another general aspect, there are provided stabilized statin particles comprising amorphous statin and stabilizers wherein one or more stabilizer is coated on the blend of one or more pharmaceutically acceptable excipients and statin. Pharmaceutically acceptable excipients may include stabilizer. The stabilizer present in the blend and coating may be same or different.

In still another general aspect, there are provided processes for preparation of stabilized statin particles comprising:
i) blending amorphous statin with one or more pharmaceutically acceptable excipients;
ii) dissolving/dispersing one or more stabilizers in solvent;
iii) fluidizing the blend of step i);
iv) spraying the solution/dispersion of stabilizers onto the fluidized blend; and
v) removing the solvent.

In another aspect, there is provided stabilized Atorvastatin particles comprising
i) a blend of amorphous Atorvastatin with alkanizing agent and chelating agent, and
ii) antioxidant,
wherein the blend of step i) is coated with antioxidant.

In still another general aspect, there are provided processes for preparation of stabilized Atorvastatin particles comprising:
i) blending amorphous Atorvastatin with alkanizing agent and chelating agent;
ii) dissolving/dispersing one or more antioxidants in solvent;
iii) fluidizing the blend of step i);
iv) spraying the solution/dispersion of antioxidants onto the fluidized blend; and
v) removing the solvent.

In a yet another aspect, there are provided stabilized statin particles comprising statin and stabilizers, wherein the statin and stabilizers are coated onto an inert carrier. The statin may be used in the amorphous or crystalline form. Where the crystalline form is used, it can be converted to amorphous form during the process of spraying to obtain the coat.

In a still further general aspect, there are provided processes for the preparation of stabilized statin particles comprising:
i) dissolving/dispersing stabilizers in solvent;
ii) dissolving/dispersing statin in suitable solvent;
iii) spraying the solution/dispersion of stabilizer and dispersion/solution of statin onto an inert carrier; and
iv) removing the solvent.

The statin may be introduced in crystalline form to the solution of step ii). In step iii) spraying of statin and stabilizer may occur simultaneously or in a sequential manner. In another general aspect, there are provided processes for the preparation of stabilized statin particles comprising:
i) dissolving/dispersing statin and stabilizers in solvent;
ii) spraying the solution/dispersion of step i) onto an inert carrier; and
iii) removing the solvent.
The statin may be introduced in crystalline or amorphous form to the solution in step i).

In yet another general aspect, there are provided processes for the preparation of stabilized statin particles comprising:
i) dissolving/dispersing stabilizers in solvent;
ii) spraying the solution/dispersion of stabilizers onto an inert carrier;
iii) powder layering amorphous statin onto the inert carrier; and
iv) removing the solvent.
Steps ii) and iii) may occur simultaneously.

In another general aspect, there are provided stabilized statin particles comprising statin and two or more stabilizers, wherein the mixture of crystalline statin and two or more stabilizers are converted into stabilized statin particles using conventional methods.

In still another general aspect, there are provided processes for the preparation of stabilized statin particles comprising:
i) dissolving crystalline statin and two or more stabilizers in suitable solvent; and
ii) spray drying or lyophilizing or solvent precipitating the solution of step i) to obtain stabilized statin particles.

### Detailed Description

The term "statin particles" as used herein means substantially amorphous statin comprising stabilizers and optionally other pharmaceutically acceptable excipients. These may be further formulated into suitable pharmaceutical compositions.

As used herein the term "statin" refers to HMG-CoA reductase inhibitors, such as pravastatin, simvastatin, lovastatin, fluvastatin, atorvastatin and rosuvastatin and their pharmaceutically acceptable salts thereof. Pharmaceutically acceptable salts of statin can be formed with metals or amines, such as alkali and alkaline earth metals or organic amines. Examples of metals which can be used as cations include sodium, potassium, magnesium, calcium, and the like. Examples of suitable amines include, for example N, N-1-dibenzylethylenediamine, chloroprocaine, choline, diethanolamine, dicyclohexylamine, ethylenediamine, N-methylglucamine, and procaine. The statin can be present in the composition from about 1% to about 50% by weight of the composition, for example from about 5 to about 40% by weight. Pharmaceutical compositions of the present invention can be used for treatment of primary hypercholesterolemia, dysbetalipoproteinemia and homozygous familial hypercholesterolemia.

The term "stabilized" as used herein means amount of total related substance is not more than 5%, particularly not more than 4.75%, when subjected to stability studies at 40°C and 75%RH for 3 months. The total related substance includes impurities such as oxo Atorvastatin, Atorvastatin diepoxide, dihydroxy epoxide and diketoepoxide. The total oxidative impurities which includes oxo Atorvastatin and Atorvastatin diepoxide is not more than 3.25% when subjected to 40°C and 75%RH for 3 months.

The term "stabilizer" as used herein means an agent that stabilizes a statin by any mechanism, for example alkanizing agent, chelating agent, photoprotectant and antioxidant. The stabilizer may be added while preparing the statin particles and/or while formulating the statin particles into suitable composition.

Examples of suitable antioxidants include, but are not limited to, butylated hydroxyanisole (BHA), sodium ascorbate, butylated hydroxytoluene (BHT), sodium sulfite, propyl gallate, tocopherol, citric acid, malic acid, and ascorbic acid. The antioxidants can be present at concentrations of, for example, up to approximately 10% by weight of the composition, for example from about 0.01% to about 5% by weight.

Examples of chelating agents include disodium EDTA, edetic acid, citric acid, and combinations thereof. The chelating agents can be present at a concentration of up to approximately 10% by weight of the composition, for example, from about 0.01 to about 5% by weight.

The term "photoprotectant" as used herein means an agent for the protection from light. Examples include metal oxides such as titanium oxide, ferric oxide or zinc oxide. The photoprotectant can be present at a concentration of up to approximately 10% by weight of the composition, for example, from about 0.01 to about 5% by weight.

The alkanizing agent as used herein can include alkali metal salt additive or alkaline earth metal salt additive. Alkali metal salt additives can be, for example, sodium carbonate, sodium hydroxide, sodium silicate, disodium hydrogen orthophosphate, sodium aluminate and other suitable alkali metal salts. In particular, the stabilizing alkali metal salt additive can be sodium carbonate or disodium hydrogen orthophosphate. Alkaline earth metal salt additives can include one or more of, for example, calcium carbonate, calcium hydroxide, magnesium carbonate, magnesium hydroxide, magnesium silicate, magnesium aluminate, or aluminum magnesium hydroxide. The amount of alkanizing agent may vary from about 1 to about 50% by weight of the composition, for example from about 1% to about 30%.

Examples of inert carriers can include, for example, nonpareil sugar spheres lactose, microcrystalline cellulose, mannitol, starch, colloidal silica, calcium phosphate and calcium carbonate. The ratio of the statin to inert carrier can be in the range of from about 1:0.1 to about 1:20, for example, from about to about 1: 1 to about 1:15.

Solutions of, stabilizers or statins or other excipients may be sprayed using equipment, known in the pharmaceutical arts, such as pan coaters (e.g., Hi-Coater available from Freund Corp. of Tokyo, Japan, Accela-Cota available from Manesty of Liverpool, U.K.), fluidized bed processors (e.g., Wurster coaters or top-sprayers available from Glatt Air Technologies of Ramsey, N.J. and from Niro Pharma Systems of Bubendorf, Switzerland) and rotary granulators (e.g., CF-Granulator, available from Freund Corp).

In case of fluidized bed processors, there are three options available with respect to the spraying pattern of the fluid. These options are top, bottom and tangential spray process.

In top spray granulation, particles are fluidized in the flow of heated air, which is introduced into the product container via a base plate. The dispersion is sprayed into the fluid bed from above against the air flow (countercurrent) by means of a nozzle. Drying takes place as the particles continue to move upwards in the air flow. Small droplets and a low viscosity of the spray medium ensure that the distribution is uniform. Since maximum product surface is exposed to the spray mist, this is a fast process and feasible for batches up to 1500 kg. This technique is usually used for coating small particles and granulation of powders.

In bottom spray granulation, the particles to be coated are kept in the product chamber and are coated with the solution with help of a Wurster design product chamber with bottom spray nozzle and partition in the middle.

The principle of operation in tangential spray is that tangential nozzles are fixed above the rotating plate so as to do different operation using the same insert. The rotating plate is a specially designed perforated plate so that the powder does not fall, and at the same time air can come through the plate, creating a fluidized effect in the processor.

The solvents used for dissolving/dispersing statin and stabilizers include methanol, ethanol, isopropanol, n-propanol, acetone, ethyl acetate, methyl ethyl ketone, MTBE (methyl-tert-butylester), hexane, cyclohexane, or aqueous solvent, water and mixtures thereof.

Conventional methods such as solvent precipitation, lyophillization, spray drying may be used to convert crystalline statin amorphous form.

Pharmaceutically acceptable excipients include binders, diluents, disintegrants, surfactants and lubricants.

Examples of suitable binders include, starch, polyvinyl pyrrolidone, hydroxypropyl cellulose, hydroxypropyl methylcellulose, and carboxymethylcellulose. Binders may be added while formulating the statin particles or/and while preparation of statin particles. In particular, binder added during preparation of statin particles may allow proper binding of drug to inert carrier.

Examples of suitable diluents include lactose, microcrystalline cellulose, corn starch, sucrose, and silicic anhydride.

Examples of suitable disintegrants include croscarmellose sodium, starch, modified starch, crospovidone and sodium starch glycolate.

Examples of suitable surfactants include, polysorbate 80, polyoxyethylene sorbitan, polyoxyethylene-polyoxypropylene copolymer, and sodium lauryl sulphate.

Examples of suitable lubricants include magnesium stearate, stearic acid, palmitic acid, talc, and aerosil.

Examples of suitable pharmaceutically acceptable glidants include colloidal silicon dioxide.

Other suitable binders, diluents, disintegrants, surfactants, lubricants, or glidants would be known to those of skill in the art.

The stabilized statin particles may be incorporated into various pharmaceutical compositions such as sachets, tablets, fast-dissolving dosage forms, chewable dosage forms, capsules, pills and caplets. These compositions can include conventional or extended release compositions. The pharmaceutical compositions may comprise additional active ingredients such amlodipine, aspirin, niacin, ezetimibe, losartan and ramipril or other combinations known to be useful in the art.

The stabilized statin particles may be prepared by various modifications. Some particular illustrative embodiments are described below.
In one of the embodiments, pharmaceutical composition may be prepared by:
i) dissolving/dispersing one or more stabilizer in solvent;
ii) blending the amorphous statin and one or more pharmaceutically acceptable excipients;
iii) fluidizing the blend of step ii);
iv) spraying the solution/dispersion of one or more stabilizer onto the fluidized blend;
v) removing the solvent; and
vi) formulating the stabilized statin particles into suitable pharmaceutical composition.
In another embodiment, pharmaceutical composition may be prepared by:
i) dissolving/dispersing antioxidant and chelating agents in solvent;
ii) blending the amorphous statin and optionally, alkanizing agent;
iii) fluidizing the blend of step ii);
iv) spraying the solution/dispersion of antioxidant and chelating agent onto the fluidized blend;
v) removing the solvent; and
vi) formulating the stabilized statin particles into suitable pharmaceutical composition.
In another embodiment, pharmaceutical composition may be prepared by:
i) dissolving/dispersing antioxidant, chelating agent and alkanizing agents in solvent;
ii) fluidizing amorphous statin;
iii) spraying the solution/dispersion of step i) onto the fluidized statin;
iv) removing the solvent; and
v) formulating the stabilized statin particles into suitable pharmaceutical composition.
In another embodiment, there is provided a process for preparation of a pharmaceutical composition comprising:
i) dissolving/dispersing one or more stabilizers in solvent;
ii) dissolving/dispersing statin in suitable solvent;
iii) spraying the solution/dispersion of statin onto an inert carrier;
iv) spraying the solution/dispersion of stabilizers onto statin particles of step iii);
v) removing the solvent of step iv); and
vi) formulating the stabilized statin particles into suitable pharmaceutical composition.
The statin may be present in crystalline or amorphous form in the solution in step ii).

In another embodiment, there is provided a process for preparation of a pharmaceutical composition comprising:
i) dissolving/dispersing one or more stabilizer in solvent;
ii) dissolving/dispersing statin in suitable solvent;
iii) spraying the solution/dispersion of dispersion of step i) and ii) simultaneously onto an inert carrier;
iv) removing the solvent of step iii); and
v) formulating the stabilized statin particles into suitable pharmaceutical composition.

In another embodiment, there is provided a process for preparation of a pharmaceutical composition comprising:
i) dissolving/dispersing one or more stabilizer in solvent;
ii) dissolving/dispersing statin along with binder in suitable solvent;
iii) spraying the solution/dispersion of dispersion of step i) and ii) simultaneously or sequentially onto an inert carrier;
iv) removing the solvent; and
v) formulating the stabilized statin particles into suitable pharmaceutical composition.

In another embodiment, there is provided a process for preparation of pharmaceutical composition comprising:
i) dissolving/dispersing antioxidant in solvent;
ii) spraying the solution/dispersion of antioxidant onto an inert carrier;
iii) blending the amorphous statin and optionally, alkanizing agent (as dry powder);
iv) powder layering the blend of step iii) onto the inert carrier as step iii);
v) removing the solvent; and
vi) formulating the stabilized statin particles into suitable pharmaceutical composition.
The step iii) and iv) may occur simultaneously in the process using tangential attachment of fluidized bed processor.

In another embodiment, there is provided a process for preparation of a pharmaceutical composition comprising:
i) dissolving crystalline statin, and two or more stabilizers in suitable solvent;
ii) spray drying the solution of step i) to obtain statin particles; and
iii) formulating the stabilized statin particles into suitable pharmaceutical composition.

In another embodiment, there is provided a process for preparation of pharmaceutical composition comprising:
i) dissolving crystalline statin, antioxidant and alkanizing agent in suitable solvent;
ii) spray drying the solution of step i) to obtain statin particles; and
iii) formulating the stabilized statin particles into suitable pharmaceutical composition.

In another embodiment, there is provided a process for preparation of a pharmaceutical composition comprising:
i) dissolving crystalline statin, antioxidant and chelating agent in suitable solvent;
ii) spray drying the solution of step i) to obtain statin particles; and
iii) formulating the stabilized statin particles into suitable pharmaceutical composition.

In another embodiment, there is provided a process for preparation of a pharmaceutical composition comprising:
i) dissolving crystalline statin, antioxidant, alkanizing agent and chelating agent in suitable solvent;
ii) spray drying the solution of step i) to obtain statin particles; and
iii) formulating the stabilized statin particles into suitable pharmaceutical composition.

In another embodiment, there is provided a process for preparation of a pharmaceutical composition comprising:
i) dissolving crystalline statin, and two or more stabilizers in suitable solvent;
ii) freeze drying the solution of step i) to obtain statin particles; and
iii) formulating the stabilized statin particles into suitable pharmaceutical composition.

In another embodiment, there is provided a process for preparation of a pharmaceutical composition comprising:
i) dissolving crystalline statin, antioxidant and alkanizing agent in suitable solvent;
ii) freeze drying the solution of step i) to obtain statin particles; and
iii) formulating the stabilized statin particles into suitable pharmaceutical composition.

In another embodiment, there is provided a process for preparation of a pharmaceutical composition comprising:
i) dissolving crystalline statin, antioxidant and chelating agent in suitable solvent;
ii) freeze drying the solution of step i) to obtain statin particles; and
iii) formulating the stabilized statin particles into suitable pharmaceutical composition.

In another embodiment, there is provided a process for preparation of a pharmaceutical composition comprising:
i) dissolving crystalline statin, antioxidant, alkanizing agent and chelating agent in suitable solvent;
ii) freeze drying the solution of step i) to obtain statin particles; and
iii) formulating the stabilized statin particles into suitable pharmaceutical composition.

In another embodiment, there is provided a process for preparation of a pharmaceutical composition comprising:
i) dissolving crystalline statin, and two or more stabilizers in suitable solvent;
ii) adding the above solution to an antisolvent;
iii) separating precipitated, statin particles form the suspension obtained in step ii); and
iv) formulating the stabilized statin particles into suitable pharmaceutical composition.

In another embodiment, there is provided a process for preparation of a pharmaceutical composition comprising:
i) dissolving crystalline statin, antioxidant and alkanizing agent in suitable solvent;
ii) adding the above solution to an antisolvent;
iii) separating precipitated, statin particles form the suspension obtained in step ii); and
iv) formulating the stabilized statin particles into suitable pharmaceutical composition.

In another embodiment, there is provided a process for preparation of a pharmaceutical composition comprising:
i) dissolving crystalline statin, antioxidant and chelating agent in suitable solvent;
ii) adding the above solution to an antisolvent;
iii) separating precipitated, statin particles form the suspension obtained in step ii); and
iv) formulating the stabilized statin particles into suitable pharmaceutical composition.

In another embodiment, there is provided a process for preparation of a pharmaceutical composition comprising:
i) dissolving crystalline statin, antioxidant, alkanizing agent and chelating agent in suitable solvent;
ii) adding the above solution to an antisolvent;
iii) separating precipitated, statin particles form the suspension obtained in step ii); and
iv) formulating the stabilized statin particles into suitable pharmaceutical composition.

Once the statin particles have been formed, several processing operations can be used to facilitate incorporation of the amorphous statin into a composition. These processing operations can include drying, granulation, and milling.

In some embodiments, the pharmaceutical composition may be prepared by wet granulation, comprising: blending stabilized statin particles, optionally with stabilizing agents and other pharmaceutically acceptable excipients; granulating that blend with a granulating fluid or solution/dispersion of binder; drying and sizing the granules; optionally blending with pharmaceutically extragranular excipients; lubricating the granules/blend; compressing the lubricated blend into suitable sized tablets; and optionally coating with film forming polymer and coating additives.

In yet another embodiment, the pharmaceutical composition may be prepared by dry granulation, comprising blending stabilized statin particles, optionally with stabilizing agents and other pharmaceutically acceptable excipients; dry granulating the blend by roller compactor or slugging; lubricating the granules/blend; compressing the lubricated blend into suitable sized tablets; and optionally coating with film forming polymer and coating additives.

In another embodiment, the pharmaceutical composition may be prepared by direct compression, comprising blending stabilized statin particles, optionally with stabilizing agents and other pharmaceutically acceptable excipients; lubricating the blend; directly compressing the lubricated blend into suitable sized tablets; and optionally coating with film forming polymer and coating additives.

Coating materials can be, for example, Opadry or Opadry AMB (aqueous moisture barrier), or other coating materials known to those of skill in the art.

While several particular embodiments have been described above, it will be apparent that various modifications and combinations of the formulations detailed in the text can be made without departing from the scope of the invention.

### Example 1

| **Ingredients** | **Qty (in mg)** |
|---|---|
| Atorvastatin Calcium amorphous | 84.39 |
| Microcrystalline Cellulose | 300.00 |
| Lactose | 618.63 |
| Sodium Carbonate | 52.00 |
| Butylated Hydroxy Anisole (BHA) | 4.58 |
| Butylated Hydroxy Toluene (BHT) | 0.40 |
| Hydroxypropyl Cellulose - low viscosity | 24.00 |
| Colloidal Silicon dioxide | 24.00 |
| Croscarmellose sodium | 72.00 |
| Sodium Lauryl Sulphate | 2.00 |
| Magnesium Stearate | 18.00 |
| Isopropyl Alcohol | q.s. |
| Tablet core weight | 1200 |
| Opadry AMB | q.s. |

1. BHA and BHT were dissolved in Isopropyl alcohol.
2. Atorvastatin Calcium and Microcrystalline cellulose were mixed together and fluidized in a fluidized bed granulator.
3. Solution of step 1 was sprayed (Inlet air temperature - 40 to 45°C and product temperature - 30 to 33°C) on fluidized statin particles of step 2 using top spray attachment in fluidized bed granulator.
4. Statin particles of step 3 were dried in fluidized bed granulator at 53°C inlet temperature.
5. Hydroxypropyl cellulose, Colloidal silicon dioxide, Croscarmellose sodium, sodium lauryl sulphate and sodium carbonate were added to statin particles of step 4 and blended together.
6. Lactose was added to blend of step 5 and blended together.
7. The blend of step 6 was lubricated and compressed into suitable size tablet.
8. Compressed tablets of step 7 were coated with Opadry.

### Example 2

| **Ingredients** | **Qty (in mg)** |
|---|---|
| Atorvastatin Calcium amorphous | 84.39 |
| Microcrystalline Cellulose | 300.00 |
| Lactose | 618.63 |
| Sodium Carbonate | 52.00 |
| Butylated Hydroxy Anisole (BHA) | 4.58 |
| Butylated Hydroxy Toluene (BHT) | 0.40 |
| Hydroxypropyl Cellulose - low viscosity | 24.00 |
| Colloidal Silicon dioxide | 24.00 |
| Croscarmellose sodium | 72.00 |
| Sodium Lauryl Sulphate | 2.00 |
| Magnesium Stearate | 18.00 |
| Isopropyl Alcohol | q.s. |
| Tablet core weight | 1200 |
| Opadry AMB | q.s. |

1. BHA and BHT were dissolved in Isopropyl alcohol.
2. Atorvastatin Calcium, Microcrystalline cellulose and a part of lactose were mixed together and fluidized in a fluidized bed granulator.
3. Solution of step 1 was sprayed (Inlet air temperature - 35 to 43°C and product temperature - 30 to 33°C) on fluidized statin particles of step 2 using top spray attachment in fluidized bed granulator.
4. Statin particles of step 3 were dried in fluidized bed granulator.
5. Hydroxypropyl cellulose, Colloidal silicon dioxide, Croscarmellose sodium, sodium lauryl sulphate and sodium carbonate were added to statin particles of step 4 and blended together.
6. The remaining part of lactose was added to blend of step 5 and blended together.
7. The blend of step 6 was lubricated using magnesium stearate and compressed into suitable size tablet.
8. Compressed tablets of step 7 were coated with Opadry.

### Example 3

| **Ingredients** | **Qty (in mg)** |
|---|---|
| Atorvastatin Calcium amorphous | 84.39 |
| Microcrystalline Cellulose | 300.00 |
| Lactose | 618.50 |
| Sodium Carbonate | 52.00 |
| Butylated Hydroxy Anisole (BHA) | 4.58 |
| Butylated Hydroxy Toluene (BHT) | 0.40 |
| Propyl Gallate | 0.20 |
| Hydroxypropyl Cellulose - low viscosity | 24.00 |
| Colloidal Silicon dioxide | 24.00 |
| Croscarmellose sodium | 72.00 |
| Sodium Lauryl Sulphate | 2.00 |
| Magnesium Stearate | 18.00 |
| Isopropyl Alcohol | q.s. |
| Tablet core weight | 1200.07 |
| Opadry AMB | q.s. |

1. BHA and BHT were dissolved in Isopropyl alcohol.
2. Atorvastatin Calcium, Microcrystalline cellulose and a part of lactose were mixed together and fluidized in a fluidized bed granulator.
3. Solution of step 1 was sprayed (Inlet air temperature - 40 to 45°C and product temperature - 30 to 33°C) on fluidized statin particles of step 2 using top spray attachment in fluidized bed granulator.
4. Statin particles of step 3 were dried in fluidized bed granulator.
5. Propyl Gallate was dissolved in Isopropyl alcohol.
6. Solution of step 5 was adsorbed on to part of lactose and was dried in a tray dryer.
7. Lactose particles of step 6 were blended with statin particles of step 4.
8. Hydroxypropyl cellulose, Colloidal silicon dioxide, Croscarmellose sodium, sodium lauryl sulphate and sodium carbonate were added to blend of step 7 and blended together.
9. The remaining part of lactose was added to blend of step 8 and blended together.
10. The blend of step 9 was lubricated using magnesium stearate and compressed into suitable size tablet.
11. Compressed tablets of step 10 were coated with Opadry.

### Example 4

| **Ingredients** | **Qty (in mg)** |
|---|---|
| Atorvastatin Calcium amorphous | 84.39 |
| Microcrystalline Cellulose | 300.00 |
| Lactose | 618.50 |
| Sodium Carbonate | 52.00 |
| Butylated Hydroxy Anisole (BHA) | 4.58 |
| Butylated Hydroxy Toluene (BHT) | 0.40 |
| Propyl Gallate | 0.20 |
| Hydroxypropyl Cellulose - low viscosity | 24.00 |
| Colloidal Silicon dioxide | 24.00 |
| Croscarmellose sodium | 72.00 |
| Sodium Lauryl Sulphate | 2.00 |
| Disodium EDTA | 10.00 |
| Magnesium Stearate | 18.00 |
| Isopropyl Alcohol | q.s. |
| Tablet core weight | 1200.07 |
| Opadry AMB | q.s. |

Procedure is same as in example 3, except at step 9 wherein Disodium EDTA is mixed with lactose and then blended with blend of step 8.

### Example 5

| **Ingredients** | **Qty (in mg)** |
|---|---|
| Atorvastatin Calcium amorphous | 84.39 |
| Microcrystalline Cellulose | 300.00 |
| Lactose | 608.50 |
| Sodium Carbonate | 52.00 |
| Butylated Hydroxy Anisole (BHA) | 4.58 |
| Butylated Hydroxy Toluene (BHT) | 0.40 |
| Hydroxypropyl Cellulose - low viscosity | 24.00 |
| Colloidal Silicon dioxide | 24.00 |
| Croscarmellose sodium | 72.00 |
| Sodium Lauryl Sulphate | 2.00 |
| Disodium EDTA | 10.00 |
| Magnesium Stearate | 18.00 |
| Isopropyl Alcohol | q.s. |
| Tablet core weight | 1199.87 |
| Opadry AMB | q.s. |

1. BHA and BHT were dissolved in Isopropyl alcohol.
2. Atorvastatin Calcium, Microcrystalline cellulose and a part of lactose were mixed together and fluidized in a fluidized bed granulator.
3. Solution of step 1 was sprayed on fluidized statin particles of step 2 using Top spray attachment in fluidized bed granulator.
4. Statin particles of step 3 were dried in fluidized bed granulator.
5. Hydroxypropyl cellulose, Colloidal silicon dioxide, Croscarmellose sodium, sodium lauryl sulphate and sodium carbonate were added to statin particles of step 4 and blended together.
6. The remaining part of lactose Disodium EDTA were blended and added to blend of step 5 and blended together.
7. The blend of step 6 was lubricated using magnesium stearate and compressed into suitable size tablet.
8. Compressed tablets were coated with Opadry.
Example 1-5 were subjected to stability studies at 40°C and 75% RH for 3 months and total related substance was found to be less than 4.75% and oxidative impurities were found to be less than 3.25%.

While several particular formulations have been described above, it will be apparent that various modifications and combinations of the formulations detailed in the text can be made without departing from the scope of the invention. For example, additional exemplary tablet formulations are given below

### Example 6

| **Ingredients** | **Qty** |
|---|---|
| Atorvastatin Calcium amorphous | 80 mg |
| Butylated Hydroxy Anisole (BHA) | 5 mg |
| Butylated Hydroxy Toluene (BHT) | 0.5 mg |
| Isopropyl Alcohol | q.s. |

Procedure
1. Dissolve BHA and BHT in Isopropyl alcohol.
2. Load Atorvastatin Calcium in Fluid bed processor and spray the solution of step 1.
3. Dry the statin particles and compress into tablets using suitable excipients

### Example 7

| **Ingredients** | **Qty** |
|---|---|
| Atorvastatin Calcium amorphous | 80 mg |
| Butylated Hydroxy Anisole (BHA) | 5 mg |
| Butylated Hydroxy Toluene (BHT) | 0.5 mg |
| Sodium Carbonate | 52 mg |
| Isopropyl Alcohol | q.s. |

Procedure
1. Dissolve BHA and BHT in Isopropyl alcohol and disperse Sodium bicarbonate in it
2. Load Atorvastatin Calcium in Fluid bed processor and spray the dispersion of Step 1.
3. Dry the statin particles and compress into tablets using suitable excipients.

### Example 8

| **Ingredients** | **Qty** |
|---|---|
| Atorvastatin Calcium amorphous | 80 mg |
| Lactose | 620 mg |
| Butylated Hydroxy Anisole (BHA) | 5 mg |
| Butylated Hydroxy Toluene (BHT) | 0.5 mg |
| Hydroxypropyl Cellulose | 12 mg |
| Isopropyl Alcohol | q.s. |

Procedure
1. Dissolve BHA, BHT and Hydroxypropyl Cellulose in Isopropyl alcohol and disperse Atorvastatin in it.
2. Load Lactose in Fluid bed processor and spray the dispersion of step 1.
3. Dry the statin particles and compressed into tablets using suitable excipients.

### Example 9

The same composition and process as in example 3, except use of crystalline atorvastatin instead of amorphous and dissolve it instead of dispersing it in step 1.

### Example 10

| **Ingredients** | **Qty** |
|---|---|
| Atorvastatin Calcium amorphous | 80 mg |
| Lactose | 620 mg |
| Sodium Carbonate | 52 mg |
| Butylated Hydroxy Anisole (BHA) | 5 mg |
| Butylated Hydroxy Toluene (BHT) | 0.5 mg |
| Hydroxypropyl Cellulose | 12 mg |
| Isopropyl Alcohol | q.s. |

Procedure
1. Dissolve BHA, BHT and Hydroxypropyl Cellulose in Isopropyl alcohol.
2. Mix Atorvastatin Calcium and Sodium Carbonate.
3. Load Lactose or suitable diluents in Fluid bed processor with tangential attachment and spray the solution of step 1 and sprinkle the blend of step 2 simultaneously using tangential attachment.
4. Dry the mixture of step 3 and compress into tablets using suitable excipients.

## Claims

1. A process for preparation of stabilized statin particles comprising:
i) dissolving/dispersing one or more stabilizers in solvent;
ii) spraying the solution/dispersion of stabilizers onto the amorphous statin; and
iii) removing the solvent.

2. The process according to claim 1 wherein statins are selected from the group consisting of pravastatin, simvastatin, lovastatin, fluvastatin, atorvastatin and rosuvastatin or pharmaceutically acceptable salts thereof.

3. The process according to claim 1 wherein stabilizer is selected from the group consisting of antioxidants, chelating agent, photoprotectant and alkanizing agent or mixtures thereof.

4. The process according to claim 3 wherein antioxidant is selected from the group consisting of butylated hydroxyanisole, sodium ascorbate, butylated hydroxytoluene, sodium sulfite, propyl gallate, tocopherol, citric acid, malic acid, ascorbic acid or mixtures thereof.

5. The process according to claim 3 wherein alkanizing agent is selected from the group consisting of sodium carbonate, sodium hydroxide, sodium silicate, disodium hydrogen orthophosphate, sodium aluminate, calcium carbonate, calcium hydroxide, magnesium carbonate, magnesium hydroxide, magnesium silicate, magnesium aluminate, aluminum magnesium hydroxide and mixtures thereof

6. The process according to claim 3 wherein chelating agent is selected from the group consisting of disodium EDTA, edetic acid, citric acid, and combinations thereof.

7. The process according to claim 1 wherein amorphous statin of step i) or statin particles are blended with one or more pharmaceutically acceptable excipients.

8. The process according to claim 7 wherein pharmaceutically acceptable excipients include stabilizers, binders, diluents, disintegrants, surfactants and lubricants.

9. The process according to claim 1 wherein the spraying of solution/dispersion of stabilizer is carried out in pan coater, fluidized bed granulator or rotary granulator.

10. The process according to claim 1 wherein the solvent is selected from the group consisting of water, methanol, ethanol, isopropanol, n-propanol, acetone, ethyl acetate, methyl ethyl ketone, methyl-tert-butylester, hexane, cyclohexane, and mixtures thereof.

11. The process according to claim 1 wherein the process further comprises the steps of:
i) blending the stabilized statin particles with pharmaceutically acceptable excipients,
ii) optionally granulating the blend of step i)
iii) lubricating the blend of step i) or granules of step ii)
iv) compressing the blend of step iii) into tablet or filling into a capsule.

12. The process according to claim 11 wherein granulation of the stabilized statin particles is carried out by dry or wet granulation technique.

13. A process for preparation of stabilized Atorvastatin particles comprising:
i) dissolving/dispersing one or more antioxidants in solvent;
ii) spraying the solution of antioxidants onto the amorphous Atorvastatin; and
iii) removing the solvent.

14. The process according to claim 13 wherein amorphous Atorvastatin of step ii) is blended with alkanizing agent and/or chelating agents before spraying the solution/dispersion of antioxidant.

15. The process according to claim 13 wherein the spraying of solution of antioxidant is carried out in Fluidized bed granulator.

16. The process according to claim 13 wherein the process further comprises the steps of:
i) blending atorvastatin particles with pharmaceutically acceptable excipients,
ii) lubricating the blend of step i),
iii) compressing the blend of step ii) into a tablet.
